Europäisches Patentamt

European Patent Office

Office européen des brevets

(19)

(11) Publication number: **0 188 047**
**B1**

(12) **EUROPEAN PATENT SPECIFICATION**

(45) Date of publication of patent specification: **31.10.90**

(51) Int. Cl.⁵: **C 12 P 19/18, C 12 N 9/10**

(21) Application number: **85300340.8**

(22) Date of filing: **18.01.85**

(54) Process for preparing fructo-oligosaccharose.

(43) Date of publication of application:
**23.07.86 Bulletin 86/30**

(45) Publication of the grant of the patent:
**31.10.90 Bulletin 90/44**

(84) Designated Contracting States:
**DE FR GB IT SE**

(56) References cited:
**GB-A-2 072 679**
**GB-A-2 105 338**
**US-A-4 276 379**

**CHEMICAL ABSTRACTS, vol. 98, no. 5, January 1983, page 538, no. 33071a, Columbus, Ohio, US; & JP - A - 57 166 981 (MEIJI SEIKA KAISHA LTD.) 14-10-1982**

(73) Proprietor: **Shinohara, Satoru**
**82 Takagsago-cho**
**Hyuga-shi Miyazaki-ken (JP)**

(72) Inventor: **Shinohara, Satoru**
**82 Takagsago-cho**
**Hyuga-shi Miyazaki-ken (JP)**

(74) Representative: **van Berlyn, Ronald Gilbert**
**23, Centre Heights**
**London, NW3 6JG (GB)**

Courier Press, Leamington Spa, England.

**EP 0 188 047 B1**

## Description

This invention relates generally to a process for preparing sweeteners, which may be conveniently utilized in the field of preparation of sweet foodstuffs. More particularly, it relates to sweeteners which may be used in so-called health foods.

This invention provides a process wherein an endotransferase is formed in an Aureobacidium sp. (Aureobacidium sp., FERM-P, 4257, ATCC 20524) and a fructooligosaccharose mainly composed of 1-kestose and nystose including sucrose bound with 1 to 3 fructose units is prepared from sucrose by the action of the thus formed endotransferase.

All references to Aureobacidium sp. herein are intended to be understood as being references to Aureobacidium sp., FERM-P, 4257, ATCC 20524.

Fructo-oligosaccharose of inulin type, in which fructose units are bound at the $\beta$-1,2 positions of sucrose, e.g. 1-kestose and nystose, are contained in certain vegetables such as onion, scallion (Allium odorum), girasole (Helianthus tuberosus L.) and oats (Avena fatua), and used as sweetners in various meals to provide them with mellow taste. However, fructo-oligosaccharose have not yet been offered in the isolated forms ready for the use thereof as individual sweeteners.

In regard of the production of oligosaccharose utilizing transferase produced by micro-organism, there has been known in the art gluco-oligosaccharose (sold under the Trade Name of Coupling Sugar) which is produced by cyclodextrin glycosyl transferase, and paratinose has also been studied. Although some research reports are found with regard to the production of nystose utilizing Apsergillus oryzae, no proposal has been made on the production of fructo-oligosaccharose, i.e. 1-kestose and nystose, from sucrose by means of Aureobacidium sp. bacteria containing fructosyl-transferase.

We have found that sucrose is not directly decomposed to glucose and fructose at the initial stage of cultivation of Auerobacidium sp., FERM-P 4257, ATCC 20524 in a culture medium containing sucrose as a sole carbon source, but 60% or more of sucrose are converted into 1-kestose and nystose wherein fructose are bound at the $\beta$-1,2 positions of sucrose. It has been further found by us that the fructo-oligosaccharose, i.e. 1-kestose and nystose, are reduced with the lapse of cultivation period so that glucose and fructose are increased with the formation of dimer and trimer of fructose, i.e. inulobiose and inulotriose. After the lapse of cultivation for 96 hours, only a trace amount of fructo-oligosaccharose is found.

A further understanding of the present invention will be had by referring to the following description concerning the initial step of the development thereof. The present invention has been conceived by us, the inventors, during our investigation for finding out biosynthetic enzymes originated from Aureobacidium sp. and utilizable for the production of polysaccharides including pururane or $\beta$-1,3-1,6-glucan. During our previous investigation referred to above, we have first judged that the increase of glucose and fructose in the culture medium is attributed to soluble invertase produced by said bacterium and have not been aware of the fact that the glucose detected at the initial stage of cultivation is the by-product glucose formed by the reaction in which fructo-oligosaccharose are produced from sucrose. Also in the initial investigation stage, the fructo-oligosaccharose which are non-reducing sugars were not detected since the saccharides in the culture medium were analyzed by means of paper chromatography for the analyses of reducing saccharides.

The clue to the development of this invention is that peaks of unknown saccharide were found at the range where peaks of trisaccharides and/or tetrasaccharides are generally found during the analysis of the cultured medium by a high speed liquid paper chromatography (HPLC) in order to study the saccharides, particularly mannitol, produced with the lapse of time in the culture medium during the cultivation for the production of polysaccharides, i.e. pururane or $\beta$-1,3-1,6-glucan. The unknown saccharides were separated and determined through liquid and gel chromatographies to ascertain that the saccharides were fructo-oligosaccharose.

In order that the invention can be more clearly understood, convenient embodiments will now be described, by way of example, with reference to the accompanying drawings, in which:

Fig. 1 is a chart of the HLPC analysis of the culture medium picked up at the initial stage of cultivation of Aureobacidium sp.

Fig. 2 is a graph showing the enzymatic action of the abcterial body containing active fructosyl-transferase enzyme.

Fig. 3 is a graph showing the change in yield of fructo-oligosaccharose as the fructosyl-transferase titre added to unit substrate (sucrose) is varied.

Fig. 4 is a graph showing the results of paper chromatography analyses of the compositions of the saccharose solutions after the enzymatic reactions as set forth in Table 8, wherein G indicates glucose, GF indicates sucrose, $GF_2$ indicates 1-kestose, $GF_3$ indicates nystose and $GF_4$ indicates 1-fructofuranosyl nystose. In Fig. 4, 10B20 and 10B4 indicates that 10 (u) of an enzyme is added to 1 gram of a substrate and reacted, respectively, for 20 hours and 4 hours.

Fig. 5 is a graph showing the yields of fructo-oligosaccharose and fructose after a sucrose solution having a low concentration of substrate (sucrose) is reacted for 60 hours.

Fig. 6 shows the chromatogram charts showing the results of liquid chromatography analyses of the saccharose solutions obtained by the enzymatic reactions set forth in Table 8, the chromatograms being obtained using the LC-4A Model available from Shimadzu Seisakusho Ltd. and using the 101N column. In

2

the charts, the peaks shows the presence of following saccharose: ①=glucose, ②=sucrose, ③=1-kestose and ④=nystose. As shown, the contents of 1-kestose and nystose amount to 60% or more.

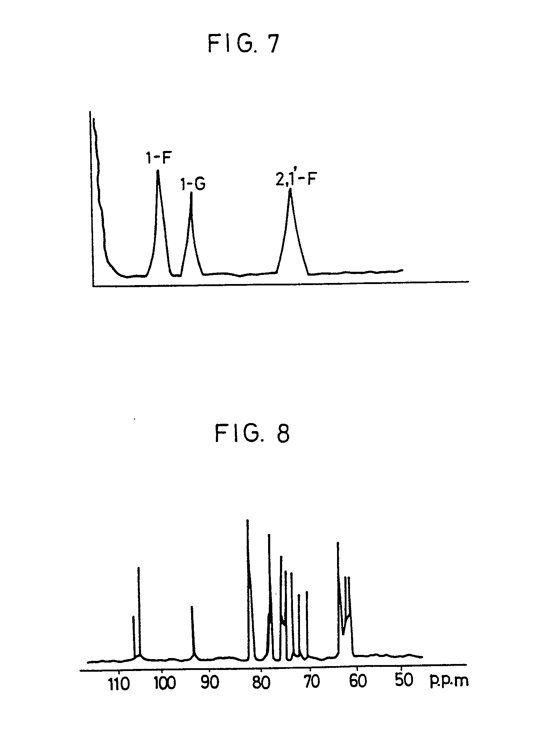

Fig. 7 is a gas chromatogram chart of an alditol acetate analysis of a methylation product of pure nystose, the pure nystose being prepared by isolating pure $GF_3$ (nystose) from a fructo-oligosaccharose solution through a gel filtration using the biogel P2 and through a liquid chromatography.

Figs. 8 and 9 show the results of $^{13}$C-NMR analyses of methylated $GF_3$ (nystose), inulin, levan and sucrose in heavy chloroform solutions. The results shown in these Figures reveal that the fructose in the fructo-oligosaccharose are bound through the inuline-type bonds, namely β-1,2 bonds.

Fig. 10 is a graph showing the changes in yield of the fructo-oligosaccharose when sucrose solutions were flown, as forming upward flows, through columns packed with fixed bacterial bodies having the fructosyl-transferase activity and fixed by β-1,3-1,6-glucan and aluminium, wherein the abscissa represents the enzyme titre (u) with which 1 gram of the substrate is allowed to contact per hour and the ordinate represents the yield of resultant fructo-oligosaccharose.

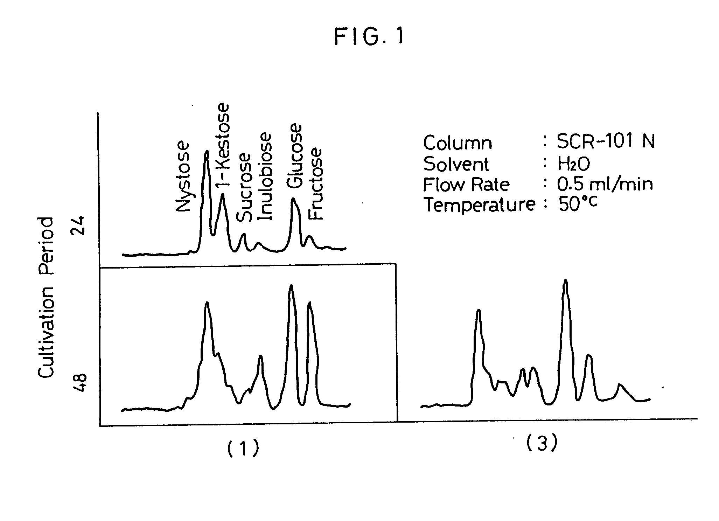

Fig. 1 shows the results of high speed liquid chromatography analyses of the cultured media at the initial stages of the cultivation of the bacterium used in the invention. According to one aspect of the invention, it is found that fructo-oligosaccharose have always been detected at the initial stage of cultivation of the bacterium of the invention by the use of sucrose as the carbon source even if the culture medium composition is varied with respect to other ingredients or conditions.

In view of the aforementioned fact, we have found that Aureobacidium sp. produces fructosyl-transferase which is a transferase for forming fructo-oligosaccharose from fructose, and we have made this invention based on that finding. We have further ascertained that the fructosyl-transferase is an endotransferase.

It should be appreciated from the foregoing that the enzyme utilized in the process of the invention may be readily produced by cultivating Aureobacidium sp. while using sucrose as the carbon source and then physically separating the bacterial body from the cultured medium. It has also been found that fructo-oligosaccharose can be readily produced by adding the thus separated bacterial body to a sucrose solution in a ratio for exhibiting a predetermined activity or titre. Since the enzyme utilized in the present invention is an endoenyzme, fructo-oligosaccharose may be produced continuously from sucrose by using the enzyme as a form of fixed bacterial body.

The present invention relates to a process for preparing fructo-oligosaccharose, wherein an Aureobacidium sp. is cultivated in a liquid culture medium to obtain a bacterial body capable of converting sucrose into fructo-oligosaccharose of inulin type mainly composed of 1-kestose and nystose or a culture medium solution containing the same which is then added to a sucrose solution or allowed to contact with a sucrose solution to convert sucrose into a fructo-oligosaccharose of inulin type mainly composed of 1-kestose and nystose by the action of fructosyl-transferase which is an endotransferase contained in said cultivated bacterial body.

The Aureobacidium sp. used in the process of the invention has been already registered as FERM-P No. 4257, ATCC No. 20524.

The bacterium referred to above is cultivated in a liquid culture medium to obtain a cultivated bacterial body or a culture medium solution containing the same.

In order to prepare a culture medium composition to be contained in the liquid culture medium, a solution containing 0.1 to 1 wt% of rice bran, 0.01 to 0.4 wt% of vitamin C and 0.5 to 5 wt% of sucrose is subjected to an aerated cultivation under agitation over a period of not less than 24 hours to obtain a cultivated solution containing active fructosyl-transferase. When the Aureobacidium sp. is cultivated in a liquid culture medium containing sucrose as the carbon source, mainly by the action of an endotransferase, *i.e.* fructosyl-transferase, for transferring one molecule of fructose from the donor sucrose to one molecule of acceptor sucrose while forming a β-1,2 bond through the reaction as set forth below, a fructo-oligosaccharose is formed as a first reaction product which acts then as an acceptor to form another fructo-oligosaccharose having an additional one mol of fructose. In either of the sequential reactions, one molecule of glucose is released.

$$2(G\text{-}F) + Enzyme \rightarrow G\text{-}F\text{-}F + G;$$

$$G\text{-}F + G\text{-}F\text{-}F + Enzyme \rightarrow G\text{-}F\text{-}F\text{-}F + G;$$

wherein G-F is sucrose, G-F-F is 1-kestose, G is glucose and G-F-F-F is nystose.

When an Aureobacidium sp. is cultivated in a liquid culture medium while using sucrose as the carbon source, the sucrose in the culture medium is converted into fructo-oligosaccharose including 1-kestose and nystose, and at the same time a bacterial body containing an endotransferase, namely fructosyl-transferase, is formed. The thus formed bacterial mass may be used as a raw enzymatic material.

However, fructosyl-transferase is not formed in the bacterial mass if other carbon sources, such as fructose, glucose or starch, are used.

Table 1 shows the cultivation conditions, the fructosyl-transferase activities and the resultant saccharide composition in the cultivated culture media when the Aureobacidium sp. are cultivated using sucrose as a sole carbon source while changing the other ingredients of the culture media.

3

As shown in Table 1, when sucrose is used as the carbon source, the fructosyl-transferase activity is confined in the bacterial cell and is not changed even if the cultivation conditions are varied.

The thus cultivated bacterial body having the fructosyl-transferase activity may be physically separated to be applied for use in the form of bacterial body, or otherwise may be directly applied for use in the form of a culture medium solution containing the bacterial body.

According to an advantageous modification of the invention, the bacterial body is physically separated from the culture medium solution and then the separated bacterial mass is fixed and used for effecting a continuous enzymatic reaction, whereby the fructo-oligosaccharose can be produced extremely easily and economically rather than using the bacterial mass in a batch process.

## TABLE 1

### Cultivation Condition

| Run No. | 1 | 2 | 3 |
|---|---|---|---|
| Carbon source | Sucrose | Sucrose | Sucrose |
| Nitrogen source | Rice bran vitamin C or zinc acetate | Powdered yeast | Powdered yeast sodium nitrate |
| pH | 5.5 | 5.5 | 6.0~6.5 |
| Temperature | 25~30°C | 25~30°C | 25~30°C |

### Fructosyl-transferase activity

| | | | |
|---|---|---|---|
| | + | + | + |

### Composition of saccharides in supernatant liquid

| | 1 | 2 | 3 |
|---|---|---|---|
| β-1,3-1,6-Glucan | + | − | − |
| α-1,4-1,6-Glucan | − | + | ± |
| Glucose | + | + | + |
| Fructose | + | + | + |
| Sucrose | + | + | + |
| 1-Ketose | + | + | + |
| Nystose | + | + | + |
| Inulobiose | + | + | + |

The so-called inclusion method using alginic acid or calcium has been studied for the fixation of general enzymes and bacterial bodies. The method originally established by us and utilized in the present invention for the fixation of active fructosyl-transferase is distinguishable from such an inclusion method, and based on the finding that β-1,3-1,6-glucan produced by the Auerobacidium sp. has a unique action of coagulating with aluminium sulfate.

More specifically, in the process of the invention, the culture solution containing β-1,3-1,6-glucan produced by the Auerobacidium sp. cultivated in a liquid culture medium is subjected to centrifugation to remove coarse particles, such as rice bran particles, and concentrated at a temperature of not higher than 60°C so that the solid concentration becomes about 3%, followed by adding the dried bacterial body having the fructosyl-transferase activity and separated from the aforementioned cultivated culture medium composition. After mixing intimately, the mixture is added with diatomaceous earth and/or kaolin so that the water content becomes about 50%, and then the mixture is extruded under pressure through a small pore into a 0.1 to 1.0% aluminium sulfate solution and dipped therein for about 30 minutes. The extruded

4

mass is then discharged from the aluminium sulfate solution, washed with water, dipped in a phosphoric buffer solution having a pH value of 6.0 to adjust the pH value thereof, and removed from the buffer solution followed by drying with hot air at a temperature of not higher than 60°C. The thus fixed bacterial body is packed in a column to have a predetermined packing density, and a sucrose solution having a sucrose concentration of 50 to 80% is flown upward through the column at a space velocity (SV) of 0.3 to 0.5 and at a temperature of 55°C to 60°C to obtain a clear and colorless oligosaccharose solution (containing 1-kestose and nystose) having an oligosaccharose content of more than 50%.

Referring back to Table 1, when the bacterium used in the invention is cultivated under the condition set forth in Run No. 1, the direct use of the cultivated solution is more rational than using the bacterial body after it is separated from the culture medium.

Specifically, when the bacterium is cultivated in a culture medium composition containing sucrose, rice bran and vitamin C, since β-1,3-1,6-glucan is produced in the culture medium solution and the β-1,3-1,6-glucan has an intensive intention to form a gel with sucrose or fructo-oligosaccharose, a solution containing fructo-oligosaccharose may be prepared through an extremely simple operation without a need of concentration or other operations only by adding sucrose to a β-1,3-1,6-glucan cultivation solution containing active fructosyl-transferase to effect enzymatic reaction thereby. This gel-forming action is not found in polysaccharides produced by other microorganisms, such as pururane, and considered to be a characteristic feature inherent to β-1,3-1,6-glucan. The solution containing β-1,3-1,6-glucan produced in accordance with the aforementioned process may be used in jam and jelly without any further processing, and may also be processed through an after-treatment to form a sweetener which provides luster, smoothness and malleability to the after-treated product.

The cultivated solution is added to a sucrose solution or allowed to contact with a sucrose solution to convert sucrose into fructo-oligosaccharose of inulin type mainly composed of 1-kestose and nystose by the action of fructosyl-transferase which is an endotransferase contained in the cultivated bacterial body. The mixing ratio of the cultivated solution to sucrose may be varied within the range of 1 (cultivated solution) to not less than 0.5 (sacrose), preferably not less than 1.0. The reaction may be carried out at a substrate concentration of not more than Brix 80, at a temperature of from 50°C to 65°C and at a pH value of from 5.0 to 8.0 while maintaining the enzyme titre to 1 gram of sucrose (substrate) within the range of not less than 5 u.

The reaction characteristics of the endotransferase will now be described. The fructosyl-transferase produced by the process as aforementioned exhibits the optimum enzymatic action within the pH range of from 5.0 to 6.0 and within the temperature range of from 55°C to 60°C. The experimental results are shown in Table 2.

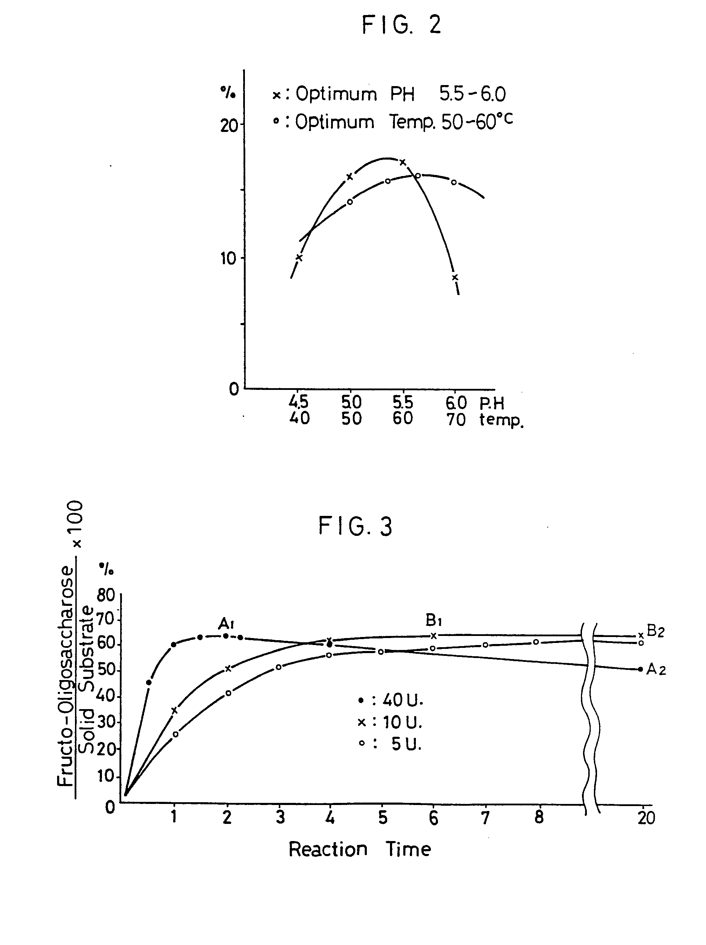

It has been ascertained that the activities of different enzymes, namely those of invertase and isomerase the presence of which are normally estimated, are not found internally of the bacterial body cultivated under the conditions set forth in Fig. 2.

The distinctive property of the sucrose used as the substrate in the process of the invention is that it acts as either a donor and an acceptor so that 1 mol of glucose and 1 mol of 1-kestose are formed from 2 mols of sucrose, and the thus produced fructo-oligosaccharose acts as an acceptor for sucrose to form a modified fructo-oligosaccharose having additional one molecular of fructose.

The basic reaction may be represented by the following equations:

$$2(G\text{-}F) + Enzyme \rightarrow (G\text{-}F\text{-}F) + G$$

$$G\text{-}F\text{-}F + G\text{-}F + Enzyme \rightarrow G\text{-}F\text{-}F\text{-}F + G$$

When sucrose acts as a donor and fructose acts as an acceptor, a dimer of fructose, *i.e.* inulobiose, and glucose are produced. This reaction may be represented by the following equation:

$$G\text{-}F + F + Enzyme \rightarrow F\text{-}F + G;$$

wherein G-F indicates sucrose, F indicates fructose, G indicates glucose and F-F indicates inulobiose. Table 2 shows saccharides, other than fructose, which may be used as acceptors.

TABLE 2

| Donor | Acceptor | | | |
|---|---|---|---|---|
| Sucrose | Frustose | Sucrose | 1-kestose | Nystose |
| | F | G-F | G-F$_2$ | G-F$_3$ |
| Reaction product | F-F | G-F$_2$ | G-F$_3$ | G-F$_4$ |
| | + | + | + | + |
| | G | G | G | G |

| Donor | Acceptor | | |
|---|---|---|---|
| Sucrose | Inulobiose | Raffinose | Melezitose |
| | F-F | GaL-G-F | G-F-F |
| Reaction product | F-F-F | GaL-G-F$_2$ | G-F-G $\mid$ F |
| | + | + | + |
| | G | G | G |

Table 2 shows that a variety of fructooligosaccharose may be produced by the utilization of the transferase activity of the enzyme of the invention by using different acceptor saccharides.

Determination of the activity or titre of the endo-enzyme will now be described. In the production of enzymes, it is required to establish a standard for the determination of the activity of produced enzyme, and the activity is generally indicated by the titre (u).

Fructosyl-transferase acts as a catalyst for preparing 1 mol of 1-kestose and 1 mol of glucose from 2 mols of sucrose. The transferase titre (u) of the enzyme may be, therefore, indicated by allowing to act the enzyme within the range where the desired enzymatic reaction occurs, namely the enzyme being used in a culture medium containing sucrose as a sole substrate, followed by determining the amount of glucose at the initial stage reaction at which nystose has not yet been detected.

The amount of glucose was determined under the conditions as described below to know the titre (u) of fructosyl-transferase. In brief, it was determined that 1 micromol Glucose=1 u.

Refined crystal sucrose was used as the substrate to prepare a culture solution containing 50% (W/V) sucrose, and the culture solution was subjected to enzymatic reaction at pH 5.5 to 5.7 and at a temperature of 55°C to 60°C for 30 minutes under an agitation at 100 rpm. The resultant glucose was determined by the Micro-Somogyi's method or high speed liquid chromatography, the amount of the reslutant glucose being calculated from the peak area when the high speed liquid chromatography was employed. The apparatus for the high speed liquid chromatography was the Model LC-4A produced and sold by Shimadzu Seisaku sho Ltd., and the used columns were SCR-101N and Bondapak Carbohydrate Analysis. Since it has been ascertained that the results of quantitative analyses through the Micro-Somogyi's method were not significantly different from those determined through the high speed liquid chromatography, subsequent analyses were conducted through the high speed liquid chromatography. The conditions for the determination of the enzymatic titres and the reaction equations are shown in Table 3.

TABLE 3
Enzyme-substrate reaction and method for
the determination of enzymatic titre

$$2G_1-_2F \xrightarrow{\text{Enzyme}} G_1-_2F_1-F+G$$

Sucrose           1-Kestose     Glucose

$G_1-_2F$(Donor)          $G_1-_2F$    $G_1-_2F_1-\frac{1}{2}F$ (1-Kestose)

$\xrightarrow{+\text{Enzyme}}$     $\rightarrow$    +   Enzyme

$G_1-\frac{1}{2}F$(Acceptor)         $G_1-\frac{1}{2}F$      G (Glucose)

Enzymatic titre: 1 u=micromol of glucose/minute

| Substrate concentration | Sucrose 50% |
|---|---|
| Temperature | 55~60°C |
| pH | 5.5~5.7 |
| Reaction time | 30 minutes |
| Determination of glucose | Micro-Somogyi's method |
|  | High speed liquid chromatography |

More than 50% of sucrose is converted into fructo-oligosaccharose by the reactions set forth above. Such a high conversion ratio is attainable by the following reasons:

The endotransferase, i.e. fructosyl-transferase, has a high activity and can be easily produced, and different enzymes including invertase and isomerase are not present.

Secondly, the bacterial body may be directly used as the enzyme source, and the enzymatic reaction may be carried out at a relatively high temperature of up to 60°C and at a relatively high substrate concentration of up to Brix 70.

The characteristic feature of the reaction of the enzyme used in the present invention resides in that sucrose acts both as a donor and an acceptor so that the fructose in the donor sucrose is transferred to the acceptor sucrose while forming the β-1,2 bond and concurrently glucose is isolated from the donor sucrose, and that the fructo-oligosaccharose formed by the first stage of the enzymatic reaction can act as an acceptor for a further reaction with sucrose.

As the results of the sequential reactions, fructo-oligosaccharose having 1 to 3 additional fructose molecules are produced.

Fructose may also act as an acceptor to react with a donor sucrose molecule to form glucose and a dimer of fructose, i.e. inulobiose.

If glucose produced by the action of the fructosyl-transferase is isomerized by an enzyme for isomerization to fructose which is then subjected to the aforementioned reaction catalyzed by the fructosyl-transferase, fructose reacts with sucrose and 1-kestose to form glucose and the dimer and trimer of fructose, i.e. inulobiose and inulotriose, whereby more than 80% of sucrose can be converted into fructo-oligosaccharose. The enzymatic reactions may be represented by the following reaction equations.

2(G-F)+Endoenzyme→G-F-F+G
G+Isomerase→F
F+G-F+Endoenzyme→F-F+G
F+G-F-F+Endoenzyme→F-F-F+G;

wherein G-F is sucrose, endoenzyme is fructosyl-transferase, G-F-F is 1-kestose, G is glucose, F is fructose, F-F is inulobiose and F-F-F is inulotriose.

In practical applications, fructosyl-transferase and isomerase (enzyme for isomerization) are allowed to act on sucrose simultaneously, or alternatively sucrose is reacted with fructosyl-transferase and then reacted with isomerase so that 80% or more of sucrose can be converted into fructo-oligosaccharose including therein fructose.

Saccharides, other than fructose, which may be used as an acceptor include 1-kestose, nystose, raffinose and melezitose. It is essential that the saccharides used as acceptors in the reaction utilized in the process of the invention have intramolecular sucrose bonds, in other words, it is essential that glucose and fructose are bound through α1-2β bond.

As has been described hereinabove, a variety of saccharides may be used as acceptors to react with donor sucrose in the presence of fructosyl-transferase. The reactions will be summarized in the Tables 4 and 5.

# EP 0 188 047 B1

## TABLE 4

| |
|---|
| Spore Bacterium (Aureobacidium sp.)→ |
| Cultivation in a Liquid Culture Medium Composition |
|    Containing Rice Bran, Vitamin C and Sucrose→ |
| Cultivation for 24 to 72 hours under the Conditions of: |
|    Aerated Fermentation under Agitation, pH 5.0~6.0, |
|    Temp.=15°C~35°C→ |
| Centrifugal Separation→ |
| Culture Medium Solution Containing Bacterial Body→ |
| Bacterial Body Containing Not Less than 0.3% of |
|    $\beta$-1,3-1,6-Glucan |

## TABLE 5

| |
|---|
| Spore Bacterium (Aureobacidium sp.)→ |
| Cultivation in a Liquid Culture Medium Composition |
|    Containing Powdered Yeast, Sodium Nitrate and Sucrose→ |
| Cultivation for 24 to 96 hours under the Conditions of: |
|    Aerated Fermentation under Agitation, pH 6.0~7.0, |
|    Temp.=15°C~35°C→ |
| Centrifugal Separation or Filtration→ |
| Filtrate of Culture Medium Solution and Filtered Bacterial Body |
|    (Containing no     (Containing Active |
|    $\beta$-1,3-1,6-Glucan)   Fructosyl-Transferase)→ |
|                   Drying |

Examples of the invention:

The present invention will now be described more specifically by referring to some examples thereof.

Example 1

Into 40 liters of city water added were 80 grams of rice bran, 80 grams of vitamin C and 2 kg of sucrose. After dissolving or suspending the additives, the pH value of the solution was adjusted to 5.5 using caustic soda, and then the solution was sterilized at 115°C under a pressure of 1.2 kg/cm$^2$ for 20 minutes. Separately, 1.5 to 2.0 liters of spore bacterium were cultivated stationarily in a 3 liter Erlenmeyer flask. After cooling the sterilized solution, the spore bacterium was inoculated and the inoculated culture medium is subjected to aerated fermentation at an air flow rate of 1/2 (v/v) to the volume of the culture medium solution and at a temperature of 25°C under an agitation at 100 rpm.

The concentration of the produced $\beta$-1,3-1,6-glucan was increased with the lapse of cultivation period up to 0.3 to 0.45%.

The cultivated solution after 48 hour cultivation contained not less than 0.3% of $\beta$-1,3-1,6-glucan and bacterial body having the fructosyl-transferase activity. 1 kg of fine granulated sugar was added to 1 liter of the cultivated solution which was subjected to enzymatic reaction at 55°C and at pH 6.0 under the agitation at 100 rpm.

Due to the inherent property of $\beta$-1,3-1,6-glucan, the sucrose solution was increased in viscosity to form a jelly, more specifically the apparent viscosity of the solution becoming higher than 500 cp/20°C, whereupon the reaction velocity of the enzymatic reaction was decreased. However, after cultivating for 16 to 20 hours, an oligosaccharose solution containing not less than 60% of oligosaccharose including 1 kestose and nystose was obtained. The thus obtained fructo-oligosaccharose solution was sterilized at 100°C for 10 minutes. The compositions of the oligosaccharose with the lapse of cultivation time are set forth in the following Table 6.

TABLE 6

| Saccharose composition | Cultivation time | | | | |
|---|---|---|---|---|---|
| | 0 | 3 | 6 | 16 | 20 |
| Glucose | — | 7.81 | 11.23 | 22.82 | 26.3 |
| Fructose | — | — | ± | 0.90 | 1.03 |
| Sucrose | 100 | 66.25 | 52.41 | 13.21 | 10.59 |
| 1-Kestose | — | 24.18 | 32.62 | 39.64 | 30.91 |
| Nystose | — | 1.76 | 3.74 | 23.43 | 30.59 |
| β-1,3-1,6-Glucan | — | 0.15 | 0.15 | 0.15 | 0.15 |

The thus produced oligosaccharose had a smooth jelly-like appearance and apparent properties similar to the white of an egg. However, since 0.15% of β-1,3-1,6-glucan was contained, the relative viscosity thereof was increased to 550 to 600 cp/20°C while starting from a sucrose solution of Brix 50, that viscosity being considerably higher than the relative viscosity, 15 to 16 cp/20°C, of a sucrose solution of Brix 50.

The saccharose compositions set forth in Table 6 were the results of high speed liquid chromatography analyses conducted by the use of the Model IC-4A (produced by Shimadze Seisakusho Ltd.) and using columns of SCR-101N and Bondapak Carbohydrate Analysis.

The molar ratio of the constitutional monosaccharides, namely the molar ratio of glucose to fructose, in the total composition of the oligosaccharose contained in the final reaction liquid was 1 to 1, which revealed that no action of any different enzymes ahd been found, in other words, no enzyme for isomerization had participated in the reaction.

The saccharose composition data obtained by the determination method adopted in the Example are proved as correct by the same fact that the molar ratio of glucose to fructose in the total oligosaccharose composition was 1 to 1.

Samples of the cultivated solution were picked up at every 24 hour intervals, subjected to centrifugal separation to isolate the bacterial bodies which were washed with water and the fructosyl-transferase activities thereof were determined. Likewise, the fructosyl-transferase activities of the culture media containing the bacterial bodies were also determined. The results are shown in Table 7.

As will be seen from Table 7, the enzyme titre (u) of the endoenzyme, namely the fructosyl-transferase activity, is not substantially changed with the lapse of time, but a slight drop is found after about 110 hours. This drop in endoenzyme titre is estimated to be attributed to dissolution of the enzyme present eternally of the cells, this estimation being supported by the fact that the amount of extracellular fructosyl-transferase is increased as seen from the result of determination of extracellular enzyme.

Almost all of sucrose contained in the culture medium is converted into fructo-oligosaccharose within the cultivation period of 24 hours, and then the amount of fructo-oligosaccharose is decreased and the saccharose composition after cultivated for 96 hours is mainly composed of glucose and fructose. The change of the saccharose composition is shown in Table 8.

TABLE 7

| Cultivation time | pH | Dried bac. body culture solution | Titre dried bac. body (u/g) | Total titre of dried bac. body/culture | Total titre culture sol. (u/l) | Total titre of dried bac. body/total titre (u/u) |
|---|---|---|---|---|---|---|
| 0 | 6.8 | 0 | | | | |
| 24 | 6.4 | 10.0 | 2720 | 27200 | 40000 | 68 |
| 48 | 6.2 | 15.6 | 2996 | 46737 | 82000 | 57 |
| 72 | 5.9 | 19.8 | 3006 | 59519 | 124000 | 48 |
| 110 | 5.8 | 26.2 | 2504 | 65604 | 164000 | 40 |
| | | | | | *220000 | |

Note:
  * Sucrose was added after the lapse of 72 hours.

**EP 0 188 047 B1**

TABLE 8

| Saccharose composition | Cultivation time | |
|---|---|---|
| | 0 | 24 |
| Fructose | — | 2.21 |
| Glucose | — | 33.43 |
| Inulobiose | — | 1.38 |
| Sucrose | 100 | 7.46 |
| 1-Kestose | — | 18.5 |
| Nystose | — | 37.0 |

Example 2

Into 1 liter of city water added were 500 grams of sucrose to prepare a 50% sucrose solution, and the pH value of the solution was adjusted to 6.0. The same spore bacterium culture solution as prepared in Example 1 was added to the sucrose solution in an amount of 5, 10 and 40 (u) for each 1 gram of substrate sucrose, and cultivated at 55°C at an agitation rate of 100 rpm. The yields of fructo-oligosaccharose at every sampling times were determined by a high speed liquid chromatography. The results of determination of glucose were well agreed with the results determined by the Micro-Somogyi's method. The results are shown in Table 9.

TABLE 9

| Titre of added enzyme/g sucrose | Reaction time | Composition of saccharose of enzymatic reaction solution | | | | | | | |
|---|---|---|---|---|---|---|---|---|---|
| | | G | GF | $GF_2$ | $GF_3$ | $GF_4$ | $GF_5$ | F | $F_2$ |
| 40A | 2 | 26.8 | 10.4 | 32.6 | 29.6 | 0.5 | | 1.3 | |
| A | 20 | 35.4 | 6.7 | 10.1 | 18.4 | 20.1 | 2.5 | 4.2 | 2.5 |
| 10B | 4 | 20.0 | 19.4 | 43.0 | 17.0 | | | 0.6 | |
| B | 20 | 27.9 | 8.8 | 21.1 | 39.6 | 0.2 | | 1.2 | 0.9 |
| 10 Brix 70 | 24 | 26.0 | 10.7 | 30.9 | 30.6 | | | 1.0 | 0.6 |

In this Table, G indicates glucose, GF indicates sucrose, $GF_2$ indicates 1-kestose, $GF_3$ indicates nystose, F indicates fructose and $F_2$ indicates inulobiose.

It is important from the industrial point of view that the reaction catalyzed by fructosyl-transferase proceeds even when the sucrose concentration is high, for example, at Brix 70.

By controlling the amount of added enzyme and the reaction time, an oligosaccharose solution containing not less than 40% of 1-kestose and an oligosaccharose solution containing not less than 39% of nystose may be produced as desired. The reactions are shown by the curves in Fig. 3.

The content of fructose is increased and inulobiose is formed when the concentration of substrate is low. The reaction may be induced by the action of isomerase or invertase which are contained in the bacterial body, or may be induced by the action of fructosyl-transferase enzyme. We, the inventors, have estimated that the reaction is induced by the action fo fructosyl-transferase when the concentration of substrate is low.

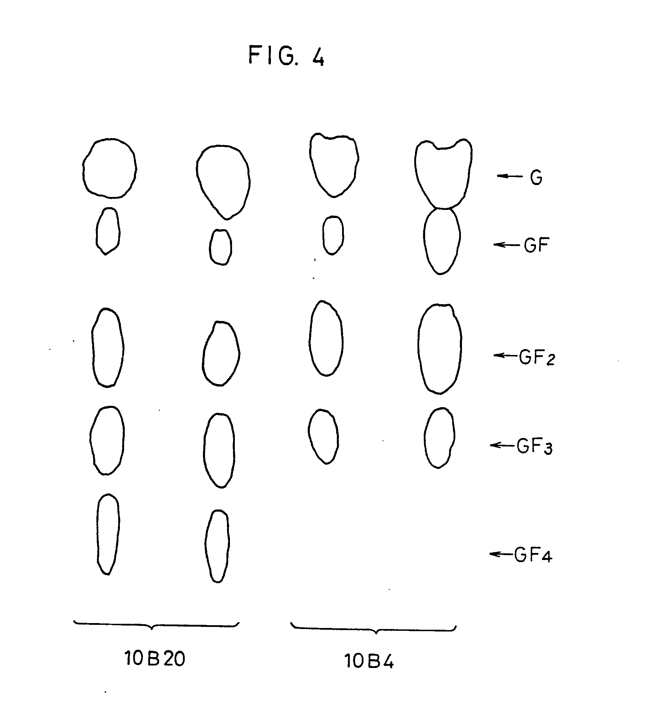

The results of paper chromatography analyses of the oligosaccharose solutions obtained by the aforementioned reactions are shown in Fig. 4.

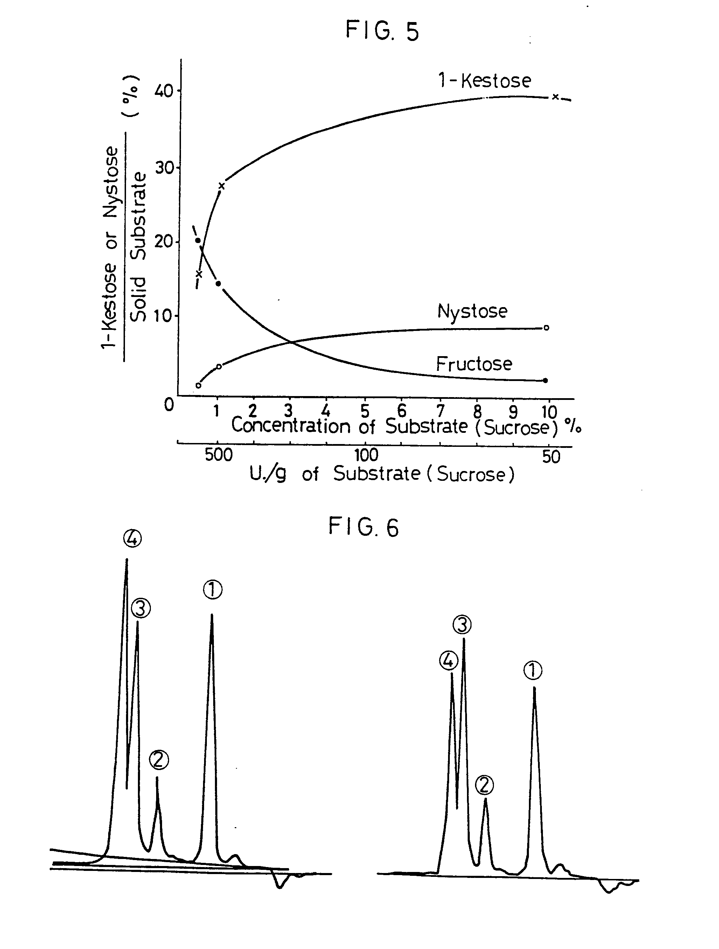

On the other hand, Fig. 5 shows the result of reaction by the enzyme used in the invention when the concentration of substrate (sucrose) is low.

However, the reactions induced by the enzyme used in the invention are characterized by the fact that they proceed even when the concentration of substrate is high, contrary to the hydrolysis reactions. For instance, oligosaccharose may be produced even when the water content is 30%. This is an important feature when applying the enzyme of the invention for the production of oligosaccharose from sucrose.

11

The solution obtained after the completion of enzymatic reactions may be heated at 100°C for 10 minutes for sterilization to obtain a product, optionally followed by filtration and decoloration.

Example 3

Into 40 liters of city water added were 400 g of sodium nitrate, 400 g of powdered yeast, 40 g of sodium primary phosphate (sodium hydrogen-phosphate) and 8 kg of sucrose to form a solution. The pH value of the solution was adjusted to 6.0 using caustic soda, and then sterilized at 115°C for 20 minutes under a pressure of 1.2 kg/cm$^2$. After cooling, 1.5 to 2.0 l of spore bacterium cultivated stationarily at 30°C in a 3 l Erlenmeyer flask was inoculated and then cultivated under aerated condition. The sample solutions were picked up, and the bacterial bodies were separated by centrifugal separation (3000 rpm/15 minutes) and the titres thereof were determined. The results are shown in Table 10.

TABLE 10

| Cultivation time | pH | Quantity of bac. body Raw bac. body (g) | | | |
|---|---|---|---|---|---|
| | | 100 ml solution | u/g | u/ml | T.u/ml |
| 0 | 6.0 | 0 | — | — | — |
| 24 | 6.0 | 2.4 | 1130 | 12.33 | 39.28 |
| 48 | 5.8 | 3.42 | 1476 | 35.40 | 81.15 |
| 72 | 5.8 | 4.04 | 1434 | 65.40 | 123.33 |
| 110 | 5.8 | 7.60 | 780 | 104.0 | 163.28 |

Since only the quantity of bacterial body is increased and no β-1,3-1,6-glucan is produced when the culture medium of this Example is used, the process of this Example is suited for using only the bacterial body as the source of fructosyl-transferase enzyme.

Although fructosyl-transferase is detected in the culture medium as an extracellular enzyme so that it may be used as extracellular fructosyl-transferase, enzymes other than fructosyl-transferase, such as invertase and isomerase, are contained in the culture medium so that the efficiency of fructosyl-transferase for producing fructo-oligosaccharose from sucrose is adversely dropped due to decomposition of sucrose and formation of fructose resulted from isomerization of glucose when the culture medium is used directly without separation. In order to obviate the aforementioned adverse side reactions, it is desirous that the bacterial body is separated for use as a source of fructosyl-transferase.

The bacterial body may be separated by conventional centrifugation and filtration methods, and the thus separated bacterial body is used after being dried.

The thus prepared bacterium having fructosyl-transferase activity was inoculated in a sucrose solution similar to that used in Example 2 to subject to enzymatic reaction. The results are similar to those as obtained in Example 2.

Example 4

360 g of the bacterial body having fructosyl-transferase activity and separated from the culture medium solution cultivated under the conditions as described in Example 3 was added to the same culture medium solution as used in Example 1. They were intimately mixed together to prepare a homogeneous solution with which admixed were 600 g of kaolin and diatomaceous earth. The admixture was then extruded under pressure through a small die pore having a diameter of 0.9 to 1.2 mm into 2 l of a 0.1% aluminium sulfate solution, and the extruded mass was dipped in the aluminium sulfate solution at normal temperature for 30 minutes. The extruded mass was then discharged from the aluminium sulfate solution, and washed with water.

Then, the washed mass was dipped in a phosphoric buffer solution maintained at pH 6.0 to adjust the pH value thereof, and then discharged from the buffer solution and dried by hot air of not higher than 60°C to obtain a fixed bacterial body. The thus obtained fixed bacterial mass was shaped to pieces each having a length of 5.0 mm and a diameter of 0.9 to 1.2 mm, and packed in a column of 1 l volume to be ready for use.

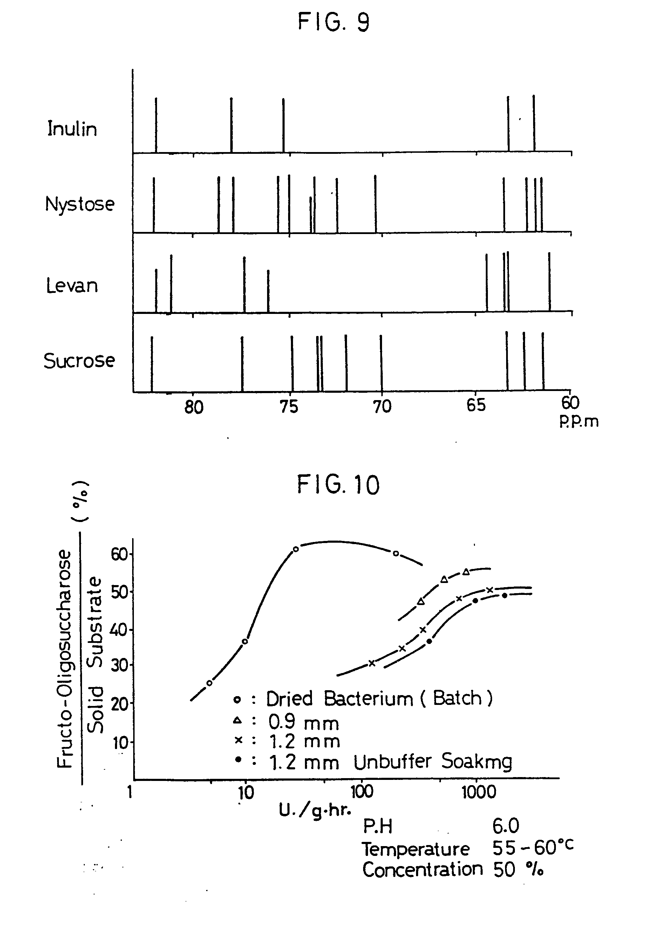

Fig. 10 shows the results obtained by flowing a 50% sucrose solution through the aforementioned column packed with the fixed bacterial body while forming an upward flow at a pH value of 6.0 and at a temperature of 60°C. The results revealed that a colorless and transparent oligosaccharose solution containing not less than 50% of oligosaccharose was prepared at a space velocity of SV=0.3 or higher. It has been also found that the conversion ratio is increased as the diameter of each fixed bacterial mass becomes smaller.

As should be appreciated from the foregoing, according to the process of the invention, sucrose can be converted into fructo-oligosaccharose of inulin type mainly composed of 1-kestose and nystose at high yield. The process of the invention may be readily applied for industrial scale production.

To comply with EPC Rule 28(1)(c), please note that the bacterium species useful in practicing the subject invention was deposited at the Fermentation Research Institute, Japanese Agency of Industrial Science & Technology on October 11, 1977 under Deposit No. 4257.

## Claims

1. A process for the preparation of a fructo-oligosaccharose, comprising the step of cultivating an Aureobacidium sp. in a liquid culture medium to obtain a cultivated bacterial body capable of converting sucrose into fructo-oligosaccharose of inulin type mainly composed of 1-kestose and nystose or a culture medium solution containing the same, and the step of adding said cultivated bacterial body or said culture medium solution containing the same to a sucrose solution to effect an enzymatic reaction therewith so that said sucrose is converted into the fructo-oligosaccharose of inulin type mainly composed of 1-kestose and nystose by the action of fructosyl-transferase which is an endotransferase contained in said cultivated bacterial mass.

2. A process for the preparation of a fructo-oligosaccharose, comprising the step of cultivating an Aureobacidium sp. in a liquid culture medium to obtain a cultivated bacterial body capable of converting sucrose into fructo-oligosaccharose of inulin type mainly composed of 1-kestose and nystose or a culture medium solution containing the same, and the step of allowing said cultivated body or said culture medium solution containing the same to contact with a sucrose solution to effect an enzymatic reaction therewith so that said sucrose is converted into the fructo-oligosaccharose of inulin type mainly composed of 1-kestose and nystose by the action of fructosyl-transferase which is an endotransferase contained in said cultivated bacterial body.

3. The process according to claim 1 or 2, wherein said liquid culture medium has a composition containing rice bran, vitamin C and sucrose, and also containing a liquid culture medium after the cultivation of bacteria containing active fructosyl-transferase and $\beta$-1,3-1,6-glucan which is obtained by an aerated cultivation under agitation for a period of not less than 24 hours.

4. The process according to claim 1 or 2, wherein said sucrose and said liquid culture medium are mixed for reaction in a mixing ratio of the liquid culture medium to the sucrose of 1 to 0.5 or more, by weight percentage.

5. The process according to any of claims 1 to 4, wherein said enzymatic reaction is effected at a temperature of from 50 to 65°C at pH 5.0 to 8.0 using a liquid culture medium having a substrate concentration of not more than Brix 80 and an enzyme titre or enzyme activity of not less than 5 u per 1 gram of the substrate sucrose, the enzyme activity unit being as defined as hereinbefore.

6. A process for the preparation of a fructo-oligosaccharose, comprising the step of fixing the active fructosyl-transferase bacteria by $\beta$-1,3-1,6-glucan obtained by the liquid cultivation of Aureobacidium sp. and aluminium sulfate or other aluminium compounds, and the step of converting sucrose into a fructo-oligosaccharose of inulin type mainly composed of 1-kestose and nystose continuously by the action of said fixed active fructosyl-transferase bacteria.

## Patentansprüche

1. Ein Prozeß zur Herstellung von einer Fructo-Oligosaccharose, bestehend aus dem Verfahrensschritt des Kultivierens eines Aureobacidium sp. in einem flüssigen Kulturmedium zur Erlangung eines kultivierten Bakterienbestandes, der in der Lage ist, Rohrzucker in Fructo-Oligosaccharose von Inulintyp umzusetzen, hauptsächlich zusammengesetzt aus 1-Kestose und Nystose, oder in einer Kulturmediumlösung, die das gleiche enthält, und dem Verfahrensschritt des Hinzufügens des genannten kultivierten Bakterienbestandes oder der Kulturmediumlösung, die denselben enthält, zu einer Rohrzuckerlösung, um eine enzymatische Reaktion mit dieser zu bewirken, so daß der Rohrzucker umgesetzt wird in die Fructo-Oligosaccharose von Inulintyp, hauptsächlich zusammengesetzt aus 1-Kestose und Nystose, durch die Wirkung von Fructosyltransferase, die eine Endotransferase ist, enthalten in der kultivierten bakteriellen Masse.

2. Ein Prozeß zur Herstellung einer Fructo-Oligosaccharose, bestehend aus dem Verfahrensschritt des Kultivierens eines Aureobacidium sp. in einem flüssigen Kulturmedium, um einen kultivierten bakteriellen Bestand zu erhalten, der in der Lage ist, Rohrzucker in Fructo-Oligosaccharose von Inulintyp, hauptsächlich zusammengesetzt aus 1-Kestose und Nystose, umzusetzen, oder in einer Kulturmediumlösung, die das gleiche enthält, und dem Verfahrensschritt, dem kultivierten Bestand oder der diesen enthaltenden Kulturmediumlösung zu ermöglichen, mit einer Rohrzuckerlösung in Kontakt zu treten, um eine enzymatische Reaktion mit dieser zu bewirken, so daß der Rohrzucker umgesetzt wird in die Fructo-Oligosaccharose des Inulintyps, hauptsächlich zusammengesetzt aus 1-Kestose und Nystose durch die Wirkung der Fructosyl-Transferase, die eine Endotransferase, enthaltend in dem kultivierten bakteriellen Körper, ist.

3. Der Prozeß nach Anspruch 1 oder 2, wobei das flüssige Kulturmedium eine Zusammensetzung hat,

13

die Reiskleie, Vitamin C und Rohrzucker enthält, und auch ein flüssiges Kulturmedium enthält nach der Kultivierung von Bakterien, enthaltend aktive Fructosyl-Transferase und β-1,3-1,6 Glucan, die erhalten wird durch eine der Luft ausgesetzte Kultivierung unter Bewegung für eine Zeitdauer von nicht weniger als 24 Stunden.

4. Der Prozeß nach Anspruch 1 oder 2, wobei der Rohrzucker und das flüssige Kulturmedium zur Reaktion gemischt werden in einem Mischverhältnis von flüssigem Kulturmedium zum Rohrzucker von 1 zu 0,5 oder mehr, gerechnet nach Gewichtsprozent.

5. Der Prozeß nach einem der Ansprüche 1 bis 4, obei die enzymatische Reaktion bei einer Temperatur zwischen 50 und 65°C bei einem pH-Wert von 5,0 bis 8,0 bewirkt wird, unter Verwendung eines flüssigen Kulturmediums, das eine Substratkonzentration von nicht mehr als Brix 80 und einen enzymatischen Titer oder enzymatische Aktivität von nicht weniger als 5 u pro 1 g des Substratrohrzuckers aufweist, wobei die enzymatische Aktivitätseinheit so definiert ist wie vorstehend.

6. Ein Prozeß zur Herstellung einer Fructo-Oligosaccharose, bestehend aus dem Verfahrensschritt des Fixierens der aktiven Fructosyl-Transferasebakterien mittels β-1,3-1,6-Glucan, erhalten durch flüssige Kultivierung von Aureobacidium sp. und Aluminiumsulfat oder andere Aluminiumverbindungen, und den Verfahrensschritt des Umsetzens von Rohrzucker in eine Fructo-Oligosaccharose von Inulintyp, hauptsächlich zusammengesetzt aus 1-Kestose und Nystose, fortlaufend durch die Wirkung der fixierten aktiven Fructosyl-Transferase-Bakterien.

**Revendications**

1. Procédé de préparation d'un fructo-oligosaccharose, comprenant l'étape de culture d'un microorganisme Aureobacidium sp. dans un milieu liquide de culture pour obtenir une masse bactérienne cultivée, capable de convertir du sucrose en fructo-oligosaccharose du type inuline, principalement composé de 1-kestose et de nystose, ou une solution de milieu de culutre contenant cette masse, et l'étape d'addition de ladite masse bactérienne cultivée ou de ladite solution de milieu de culture contenant cette masse à une solution ce sucrose, pour effectuer avec une réaction enzymatique de façon que ledit sucrose soit converti en le fructo-oligosaccharose du type inuline, principalement composé de 1-kestose et de nystose, par l'action d'une fructosyl-transférase qui est une endotransférase contenue dans ladite masse bactérienne cultivée.

2. Procédé pour la préparation d'un fructo-oligosaccharose, comprenant l'étape de culture d'un microorganisme Aureobacidium sp. dans un milieu de culture liquide pour obtenir une masse bactérienne cultivée capable de convertir du sucrose en fructo-oligosaccharose de type inuline, princiaplement composé de 1-kestose et de nystose, ou d'une solution de milieu de culture contenant cette masse, et l'étape consistant à laisser ladite masse cultivée ou ladite solution de milieu culture contenant cette masse en contact avec une solution de sucrose, pour effectuer avec une réaction enzymatique de telle sorte que ledit sucrose soit converti en le fructo-oligosaccharose du type inuline, principalement composé de 1-kestose et de nystose, par l'action d'une fructosyl-transférase qui est une endotransférase contenue dans la masse bactérienne cultivée.

3. Procédé conforme à la revendication 1 ou 2, dans lequel ledit milieu de culture liquide présente une composition contenant du son de riz, de la vitamine C et du sucrose, et contenant également un milieu de culture liquide après culture bactérienne contenant une fructosyl-transférase active et du β-1,3-1,6-glucane, que l'on obtient par culture aérée sous agitation pendant une période d'au moins 24 heures.

4. Procédé conforme à la revendication 1 ou 2, dans lequel ledit sucrose et ledit milieu de culture liquide sont mélangés pour la réaction en un rapport de mélange du milieu de culture liquide au sucrose de 1 à 0,5 ou plus, en pourcentage pondéral.

5. Procédé conforme à l'une quelconque des revendications 1 à 4, dans lequel ladite réaction enzymatique est effectuée à une température de 50 à 65°C, à un pH de 5,0 à 8,0 avec un milieu de culture liquide présentant une concnetration de substrat d'au plus 80° Brix et un titre d'enzyme ou une activité d'enzyme d'au moins 5 u par gramme de substrat sucrose, l'unité d'activité d'enzyme étant telle que définie plus haut.

6. Procédé pour la préparation d'un fructo-oligosaccharose, comprenant l'étape de fixation de bactéries à fructosyl-transférase active par le β-1,3-1,6-glucane obtenu par culture liquide de Aureobacidium sp. et par du sulfate d'aluminium ou d'autres composés de l'aluminium, et l'étape de conversion du sucrose en un fructo-oligosaccharose de type inuline, principalement composé de 1-kestose et de nystose, en continu par l'action de ladite bactérie fixée à fructosyl-transférase active.

FIG. 1

FIG. 2

FIG. 3

# FIG. 4

←— G

←—GF

←—GF$_2$

←—GF$_3$

←—GF4

10 B 20          10 B 4

EP 0 188 047 B1

FIG. 5

FIG. 6

FIG. 7

FIG. 8

## FIG. 9

Inulin

Nystose

Levan

Sucrose

80    75    70    65    60
P.P.m

## FIG. 10

Fructo-Oligosuccharose / Solid Substrate ( % )

○ : Dried Bacterium ( Batch )
△ : 0.9 mm
× : 1.2 mm
● : 1.2 mm Unbuffer Soakmg

1    10    100    1000
U./g·hr.

P.H            6.0
Temperature    55 – 60°C
Concentration  50 %

6